# EUROPEAN PATENT APPLICATION

(11) **EP 4 338 681 A1**
(43) Date of publication of application: **20.03.2024**
(21) Application number: 22807111.4
(22) Date of filing: 04.03.2022
(51) Int. Cl.: A61B 8/08

(54) **MONITORING DEVICE**

(30) Priority: 11.05.2021 JP 2021080399
(71) Applicant: Niterra Co., Ltd., Nagoya-shi, Aichi 461-0005 (JP)
(72) Inventor: HASEGAWA Hiroko, Nagoya-shi, Aichi 467-8525 (JP); TAKAGI Yutaka, Nagoya-shi, Aichi 467-8525 (JP); TSUBOI Yoshiki, Nagoya-shi, Aichi 467-8525 (JP); NADANAMI Norihiko, Nagoya-shi, Aichi 467-8525 (JP)
(74) Representative: Grünecker Patent- und Rechtsanwälte PartG mbB
(86) International application number: PCT/JP2022/009310
(87) International publication number: WO 2022/239404

(57) **Abstract**

A monitoring device (10) includes an emitting part (20) that emits an ultrasonic wave, a reception part (20) that receives a reflected wave, and a detection part (30). When the emitting part (20) emits the ultrasonic wave to a bottom of bladder (112) of a subject (100), the reception part (20) receives a reflected wave from the bottom of bladder (112), the reflected wave being based on the ultrasonic wave from the emitting part (20). The detection part (30) detects, based on a result of reception by the reception part (20), a predetermined movement of the bottom of bladder (112) or an index indicating a moving state of the bottom of bladder (112).

## Description

### Technical Field

The present invention relates to a monitoring device.

### Background Art

PTL 1 discloses an example of a urination prediction device. The urination prediction device disclosed in PTL 1 includes a plurality of ultrasonic sensors and a server group. The plurality of ultrasonic sensors each transmit an ultrasonic wave to the body of a subject and detect the bladder. The server group estimates urination timing based on a bladder inflation speed obtained from the results of detection by the plurality of ultrasonic sensors.

### Citation List

### Patent Literature

PTL 1: International Publication No. 2018/185904

### Summary of Invention

### Technical Problem

The urination prediction device in PTL 1 is a device that detects the inflation speed of the bladder to predict urination timing. However, it is not possible for the urination prediction device in PTL 1 to evaluate accurately the movement of a bottom of bladder in a short period.

The present invention has been made to solve the above-described problem and aims to provide a monitoring device capable of evaluating more accurately the movement of the bottom of bladder in a short period.

### Solution to Problem

A monitoring device according to one of the present inventions has an emitting part that emits an ultrasonic wave into a body of a subject.

The monitoring device includes:
a reception part that receives a reflected wave based on the ultrasonic wave reflected on a bottom of bladder of the subject; and
a detection part that detects, based on a result of reception by the reception part, a predetermined movement of the bottom of bladder or an index indicating a moving state of the bottom of bladder.

The monitoring device described above is capable of emitting an ultrasonic wave to a bottom of bladder and detecting a predetermined movement of the bottom of bladder or detecting an index indicating a moving state of the bottom of bladder. The monitoring device described above does not focus on and monitor only the inflation or the shrinkage of the bladder as a whole, but can focus on the bottom of bladder and monitor the movement thereof. Therefore, even in a short period, when the bottom of bladder moves, the monitoring device can easily evaluate the movement more accurately. The monitoring device described above is thus useful in verifying a state of a motion near the pelvic floor muscles.

The monitoring device described above may include an output part that outputs information indicating a result of detection by the detection part.

When the monitoring device thus includes the output part, information regarding the evaluation of the movement of the bottom of bladder becomes available, and convenience is further enhanced.

In the monitoring device described above, the information described above may include at least one of the following: a count of occurrence of the predetermined movement, a moving distance of the bottom of bladder, a moving speed of the bottom of bladder, whether or not the predetermined movement has occurred, and a period of time in which the moving state of the bottom of bladder is maintained.

When the information output by the output part thus includes at least one of the following: "a count of occurrence of the predetermined movement", "a moving distance of the bottom of bladder", "a moving speed of the bottom of bladder", "whether or not the predetermined movement has occurred", and "a period of time in which the moving state of the bottom of bladder is maintained", the moving state of the bottom of bladder is evaluated based on objective reference, and the evaluation information thereof can be used effectively.

In the monitoring device described above, the output part may include a transmission part that transmits the information to an external apparatus.

When the transmission part is thus configured to be able to transmit the information to the external apparatus, the information can be used effectively in the external apparatus.

The monitoring device described above may include an attaching unit to be attached to the subject. The output part may include a notification part provided in the attaching unit. The notification part may output the information by at least one of sound output or displaying.

When the attaching unit to be attached to the subject is thus provided with the notification part, and the notification part outputs the information by at least one of sound output or displaying, the subject can recognize the information more easily.

In the monitoring device described above, the emitting part may include a plurality of ultrasonic generation elements. When ultrasonic waves are emitted from two or more ultrasonic generation elements included in the plurality of ultrasonic generation elements to a bladder of the subject, and any of the ultrasonic waves is emitted to the bottom of bladder, the reception part may receive reflected waves from the bladder based on the respective ultrasonic waves.

The monitoring device described above is capable of receiving and using not only one reflected wave from the bottom of bladder but also another reflected wave from the bladder. Therefore, evaluation can be performed from more various angles, and the movement of the bottom of bladder is likely to be evaluated more accurately.

In the monitoring device described above, the emitting part may be configured to emit the ultrasonic waves to the bottom of bladder and an inner side wall of bladder of the subject when the emitting part is disposed in a predetermined positional relationship with the subject. The reception part may be configured to receive reflected waves from the bottom of bladder and the inner side wall of bladder based on the respective ultrasonic waves.

The monitoring device described above is capable of evaluating not only the movement of the bottom of bladder but also the movement of the inner side wall of bladder. With the monitoring device as described above, it is possible to verify, for example, "a movement such that the bottom of bladder moves largely in a state where the movement of the inner side wall is little or there is no movement of the inner side wall of bladder" or the like can also be verified. It is thus considerably useful in evaluating a state of motion near the pelvic floor muscles more accurately.

In the monitoring device described above, the emitting part may be configured to emit the ultrasonic waves to the bottom of bladder of the subject when the emitting part is disposed in a predetermined positional relationship with the subject. The reception part may be configured to receive reflected waves from the bottom of bladder based on the respective ultrasonic waves.

The monitoring device described above is capable of evaluating the movement of the bottom of bladder more accurately by using a plurality of reflected waves. For example, in the case of the occurrence of a detection malfunction such that any of the reflected waves does not accurately reflect the movement of the bottom of bladder, it is possible to enhance the accuracy in evaluating the movement of the bottom of bladder by using other reflected waves. Alternatively, if there is a difference in movement between the parts generating the respective reflected waves, information on the movement of each part can be collected and evaluated.

### Advantageous Effects of Invention

The monitoring device according to one aspect of the present invention can evaluate the movement of the bottom of bladder in a short period.

### Brief Description of Drawings

[Fig. 1] Fig. 1 is a block diagram schematically illustrating the electrical configuration of a monitoring device of a first embodiment.
[Fig. 2] Fig. 2 is a view illustrating a state where the monitoring device of the first embodiment is attached to a subject.
[Fig. 3] Fig. 3 is a view conceptually illustrating a state where the monitoring device of the first embodiment emits an ultrasonic wave to the bladder.
[Fig. 4] Fig. 4 is a flowchart illustrating the flow of control performed by the monitoring device of the first embodiment.
[Fig. 5] Fig. 5 is a graph illustrating an example of a waveform obtained by receiving the reflected wave by a reception part.
[Fig. 6] Fig. 6 is a graph illustrating a waveform obtained by receiving the reflected wave by the reception part in an example different from the example in Fig. 5.
[Fig. 7] Fig. 7 is a block diagram schematically illustrating the electrical configuration of a monitoring device of a second embodiment.
[Fig. 8] Fig. 8 is a view conceptually illustrating a state where the monitoring device of the second embodiment emits ultrasonic waves to the bladder.
[Fig. 9] Fig. 9 is a view conceptually illustrating a state where an ultrasonic wave is emitted to the bladder with the monitoring device of the first embodiment attached to a subject different from the subject in Fig. 3. Description of Embodiments

### 1. First Embodiment

### 1-1. Basic Configuration of Monitoring Device 10 and Components

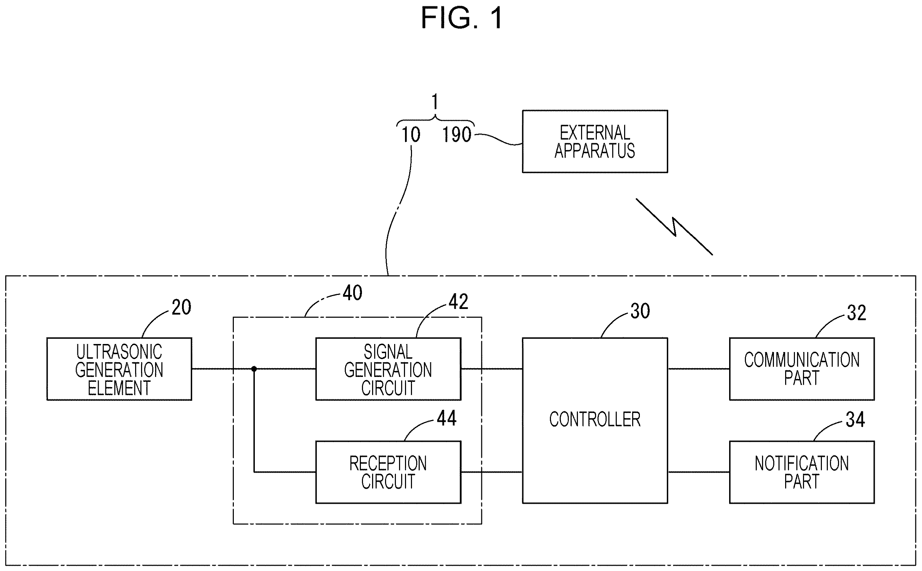

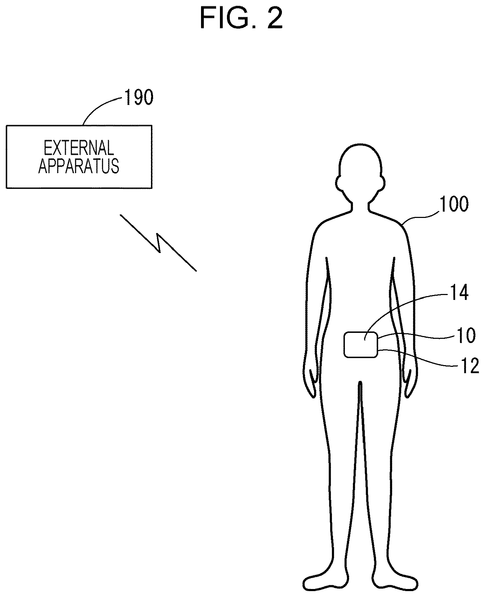

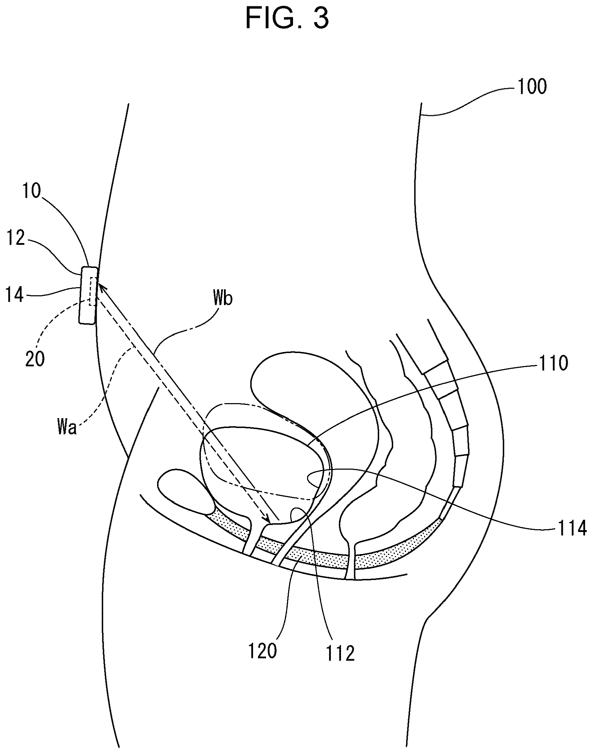

Fig. 1 illustrates a monitoring device 10 and components according to a first embodiment. The monitoring device 10 illustrated in Fig. 1 is attached to the human body of a subject 100 as illustrated in Fig. 2 and monitors the inside of the body of the subject 100. Specifically, as illustrated in Fig. 3, the monitoring device 10 functions as a bottom-of-bladder monitoring device that emits an ultrasonic wave into the body of the subject 100 and that monitors the movement of a bottom of bladder 112 of the subject 100 by using the ultrasonic wave.

As illustrated in Fig. 1, the monitoring device 10 includes an ultrasonic generation element 20, a controller 30, a transmission/reception circuit 40, a communication part 32, and a notification part 34. The monitoring device 10 is configured to be able to communicate with an external apparatus 190. In the example in Fig. 1, a monitoring system 1 includes the monitoring device 10 and the external apparatus 190.

The monitoring device 10 of the first embodiment includes a housing 14, components integrally provided together with the housing 14 (the ultrasonic generation element 20, the controller 30, the transmission/reception circuit 40, the communication part 32, the notification part 34, and other components as illustrated in Fig. 1), and a fixing part for attaching the housing 14 to the subject 100. The components (the ultrasonic generation element 20, the controller 30, the transmission/reception circuit 40, the communication part 32, the notification part 34, and other components) are accommodated in the housing 14. In the example in Fig. 3, an attaching unit 12 is composed of the housing 14.

In the monitoring device 10 of this embodiment, the housing 14 and the components (the ultrasonic generation element 20, the controller 30, the transmission/reception circuit 40, the communication part 32, the notification part 34, and other components) are integrally provided, but it is not limited to this. Among these components, the controller 30, the transmission/reception circuit 40, the communication part 32, the notification part 34, and other components may be provided separately from the housing 14.

The illustration of the fixing part is omitted in Figs. 2 and 3. The fixing part is a fastening tool (such as a clip, a belt, or tape) for fastening the housing 14 onto the human body of the subject 100 or an object (such as clothes) fit to the subject 100. It suffices that the fixing part is configured to enable the monitoring device 10 to be attached to the subject 100 in a predetermined disposition state. The predetermined disposition state is a disposition state where an ultrasonic wave discharged from the ultrasonic generation element 20 is applied to the bottom of bladder 112 (Fig. 3). The bottom of bladder 112 is a portion in the human body of the subject 100 that is composed of a predetermined part included in an inner wall portion of a bladder 110 (a bladder bottom position) and tissues consisting of pelvic floor muscles.

The ultrasonic generation element 20 is an element having a function as an ultrasonic element for transmission and a function as an ultrasonic element for reception. The ultrasonic generation element 20 corresponds to an example of an emitting part and has a function of emitting the ultrasonic wave. The ultrasonic generation element 20 corresponds to an example of a reception part and has a function of receiving a reflected wave (ultrasonic wave) based on the ultrasonic wave emitted by the emitting part, reflected on an object, and returned therefrom.

In the example in Fig. 1, the ultrasonic generation element 20 is composed of a piezo-electric element. When a transmission signal (driving signal) at a predetermined resonant frequency is applied, an ultrasonic wave based on flexural vibration of a vibrator is radiated. The ultrasonic generation element 20 discharges the ultrasonic wave corresponding to the transmission signal in a predetermined direction. If the ultrasonic wave discharged from the ultrasonic generation element 20 in the predetermined direction is reflected on the object present in the predetermined direction, a reflected wave caused by the reflection is received by the ultrasonic generation element 20 (wave receiving). The ultrasonic generation element 20 converts the reflected wave (ultrasonic wave) received by itself into an electric signal. A method for calculating a distance based on a received waveform will be described later.

The transmission/reception circuit 40 includes a signal generation circuit 42 and a reception circuit 44. In the monitoring device 10, the ultrasonic generation element 20 and the transmission/reception circuit 40 function as an ultrasonic sensor.

The signal generation circuit 42 includes an electric circuit that drives the ultrasonic generation element 20 and thereby causes the ultrasonic generation element 20 to generate an ultrasonic wave. The signal generation circuit 42 includes, for example, oscillation circuitry that generates an alternating current signal and an amplifier circuit that supplies the ultrasonic generation element 20 with the amplified signal generated by amplifying the alternating current signal generated by the oscillation circuitry. The signal generation circuit 42 drives the ultrasonic generation element 20 in response to an instruction from the controller 30.

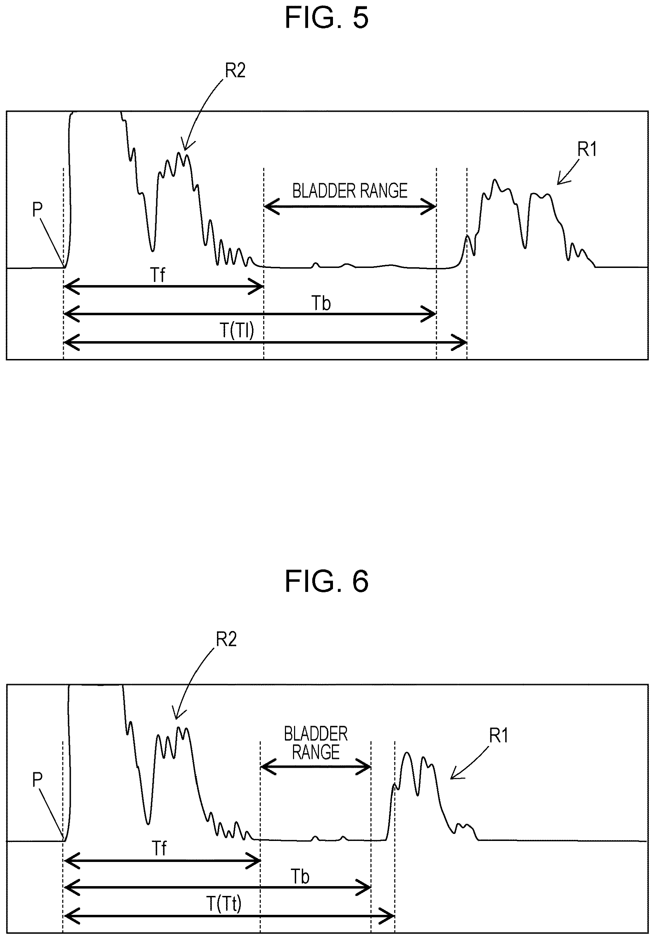

The reception circuit 44 includes, for example, an amplifier circuit, a lowpass filter circuit, an AD conversion circuit, and other components. The amplifier circuit generates an amplified signal by amplifying a reception signal generated by the ultrasonic generation element 20 when the ultrasonic generation element 20 receives an ultrasonic wave (for example, a reflected wave). The lowpass filter circuit performs lowpass filter processing for removing a high-frequency component from the amplified signal generated by the amplifier circuit. The AD conversion circuit converts, into digital data, a signal from which the high-frequency component is removed by the lowpass filter circuit (a signal indicating the reception result) (see Figs. 5 and 6), and supplies the digital data to the controller 30. The reception circuit 44 herein described is merely an example, and it suffices that the reception circuit 44 is configured to be able to supply a signal indicating a waveform of the signal received by the reception part to the controller 30. For example, the reception circuit 44 may be configured to convert the amplified signal generated by the amplifier circuit into digital data and then supply the digital data to the controller 30.

The controller 30 functions as a controller that controls the overall operation of the monitoring device 10. The controller 30 is an information processing apparatus having various information processing functions such as a computing function, a storing function, an input and output function, and a read-out function. The controller 30 includes a controller device such as a micro controller unit (MCU). The controller 30 may also include a memory, a timer, and the like.

The communication part 32 is a device that performs communication with an external apparatus by a publicly known wireless communication method or wired communication method. For example, the communication part 32 is capable of performing wireless communication with the external apparatus 190 by a publicly known wireless communication method. The communication part 32 transmits, in cooperation with the controller 30, various pieces of information to the external apparatus 190. The communication part 32 also receives, in cooperation with the controller 30, various pieces of information from the external apparatus 190.

The external apparatus 190 is an information processing apparatus such as a smartphone, a tablet terminal, or a personal computer. The external apparatus 190 includes a display device (such as an image display), a sound output device (such as a speaker), a memory device, a communication device, and other devices. The external apparatus 190 performs communication with the monitoring device 10. The external apparatus 190 has a function of receiving information from the monitoring device 10 and a function of transmitting information to the monitoring device 10.

The notification part 34 includes a display device that displays information such as a figure or a symbol. The notification part 34 further includes a sound output device such as a speaker.

### 1-2. Operations of Monitoring Device 10

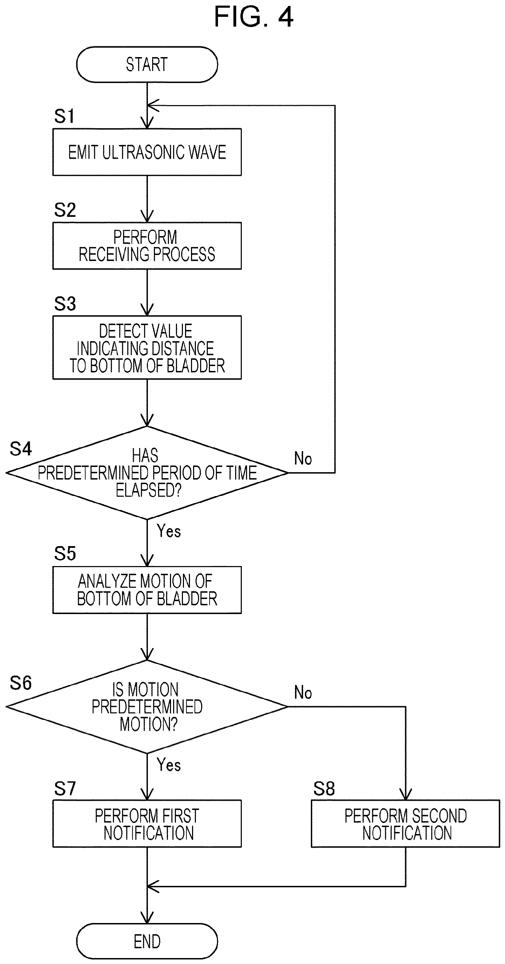

The monitoring device 10 is capable of performing control illustrated in Fig. 4. A memory provided in or outside the controller 30 in the monitoring device 10 stores a program for performing the control in Fig. 4. If a predetermined starting condition is fulfilled, the controller 30 runs the program and performs the control in Fig. 4 in accordance with the program. The "predetermined starting condition" may be a predetermined operation performed on an operation part (not illustrated) provided in the monitoring device 10, or may be another condition established (such as power-on or arrival of predetermined reservation time).

In response to the establishment of the "predetermined starting condition", the controller 30 starts the control in Fig. 4. The controller 30 first performs processing in step S1. The processing in step S1 is processing for causing the ultrasonic generation element 20 to generate an ultrasonic wave. The controller 30 gives an instruction to the signal generation circuit 42 and causes the signal generation circuit 42 to perform an operation to drive the ultrasonic generation element 20 (an operation to cause the ultrasonic generation element 20 to generate an ultrasonic wave).

After step S1, the controller 30 performs processing in step S2. The processing in step S2 is processing for acquiring a "signal indicating the result of reception by the ultrasonic generation element 20", the "signal" being provided from the reception circuit 44 over a certain length of time (predetermined monitoring time) immediately after the ultrasonic generation element 20 is caused to generate the ultrasonic wave in the processing in step S1. The "signal indicating the result of reception by the ultrasonic generation element 20" is illustrated in, for example, Figs. 5 and 6. The "signal indicating the result of reception by the ultrasonic generation element 20" illustrated in Figs. 5 and 6 is a signal in which a high-frequency component is removed from the amplified signal obtained by amplifying the reception signal generated by the ultrasonic generation element 20 by receiving the ultrasonic wave.

After step S2, the controller 30 performs processing in step S3. The processing in step S3 is processing for detecting a distance to the bottom of bladder 112 based on the "signal indicating the result of reception by the ultrasonic generation element 20", the "signal" being acquired in the processing in step S2. In this embodiment, as illustrated in Fig. 3, in a case where the ultrasonic generation element 20 (emitting part) emits an ultrasonic wave Wa to the bottom of bladder 112 of the subject 100, the ultrasonic generation element 20 (reception part) receives a reflected wave Wb (ultrasonic wave) from the bottom of bladder 112. The reflected wave Wb is based on the ultrasonic wave Wa from the ultrasonic generation element 20. In such a case, the "signal indicating the result of reception by the ultrasonic generation element 20" includes time information indicating the distance from the ultrasonic generation element 20 to the bottom of bladder 112.

Specifically, the controller 30 analyzes the "signal indicating the result of reception by the ultrasonic generation element 20" acquired in step S2, and detects an elapsed time from a "predetermined reference time" until a "time when the reflected wave from the bottom of bladder 112 is detected".

Figs. 5 and 6 illustrate the reflected waveform (the waveform of the signal indicating the result of reception by the ultrasonic generation element 20) at the time when the ultrasonic wave is emitted from the ultrasonic generation element 20 to the bladder 110 with the monitoring device 10 being disposed on the abdominal region of the subject 100 in a positional relationship in which the ultrasonic wave reaches the bottom of bladder 112, as illustrated in Fig. 3. Fig. 5 illustrates reflected waveforms in a normal state where the subject 100 loosens pelvic floor muscles 120 (the state represented by the solid line in Fig. 3). Fig. 6 illustrates reflected waveforms in a state where the subject 100 exerts his/her strength in such a manner as to tense the pelvic floor muscles 120 (the state represented by the alternate long and two short dashes line in Fig. 3). In Figs. 5 and 6, a reflected waveform R2 which corresponds to a part closer to the surface of the human body of the subject 100 (the human body surface near the monitoring device 10) is a reflected waveform caused by muscles, subcutaneous fat, the inner front wall of the bladder, and the like. In Fig. 5, a reflected waveform R1 which corresponds to a part farther from the surface of the human body of the subject 100 (the human body surface near the monitoring device 10) represents a reflected wave from the bottom of bladder 112 and the body tissues behind the bottom of bladder 112. Since the bladder 110 has urine, there is almost no reflection from the bladder 110, so that an amplitude indicating the reflection intensity is low.

In the processing in Fig. 3, time when the reflected wave rises for the first time is defined as a reference time P in the reflected waveform (the waveform of the signal indicating the result of reception by the ultrasonic generation element 20) as shown in Figs. 5 and 6. A period of time from the reference time P to the termination time of the reflected waveform R2 (elapsed time) is denoted by Tf, and a period of time from the reference time P to the rising time of the reflected waveform R1 (elapsed time) is denoted by Tb. A waveform in a range from the elapse of the period of time Tf after the reference time P to the elapse of the period of time Tb is defined as a waveform in the range corresponding to the inside of the bladder 110. "The waveform in the range corresponding to the inside of the bladder 110" is thus defined, and the level of a peak (amplitude level) in "the waveform in the range corresponding to the inside of the bladder 110" is defined as a reference peak level. Then, in the reflected waveform R1 having a plurality of peaks at an amplitude level that is more than two times as high as the reference peak level, the peak closest to the reference time P (the earliest peak) is defined as a peak representing the position of the bottom of bladder 112 (bladder bottom peak). A period of time T from the reference time P to the bladder bottom peak is used as a "value indicating the distance from the monitoring device 10 to the bottom of bladder 112". In the waveform in Fig. 5, the period of time T is denoted by Tl. In the waveform in Fig. 6, the period of time T is denoted by Tt. In step S3, the value indicating the distance from the monitoring device 10 to the bottom of bladder 112 (period of time T) is detected in this manner. As illustrated in Fig. 5, the amplitude of the bladder bottom peak becomes low in the state where the bottom of bladder 112 is lowered by loosening the pelvic floor muscles 120, and as illustrated in Fig. 6, the amplitude of the bladder bottom peak becomes high in the state where the bottom of bladder 112 is raised by tensing the pelvic floor muscles 120. Accordingly, the movement of the bottom of bladder 112 may be judged based on a change of the amplitude of the bladder bottom peak in the reflected waveform R1. For example, if the amplitude of the bladder bottom peak has changed by an amount equal to or exceeding a threshold, it may be judged that the bottom of bladder 112 has moved.

After the processing in step S3, the controller 30 performs the processing in step S4. The processing in step S4 is processing for judging whether a predetermined period of time has elapsed since the start of the control in Fig. 4. If a predetermined period of time has not elapsed since the start of the control in Fig. 4, the controller 30 moves the processing back to step S1. If a predetermined period of time has elapsed since the start of the control in Fig. 4, the controller 30 moves the processing forward to step S5.

In step S5, the controller 30 analyzes the motion of the bottom of bladder 112 based on the results of the processing in step S3 repeated a plurality of times. In step S6, the controller 30 judges whether a predetermined motion is performed (a reciprocating action of the bottom of bladder 112 that is performed at a speed equal to or higher than a predetermined moving speed within the predetermined period of time and represented by a value exceeding a certain value (a rise represented by a value exceeding a certain value and a drop represented by a value exceeding a certain value)). In the control in Fig. 4, the "value indicating the distance from the monitoring device 10 to the bottom of bladder 112" can be acquired every time the processing in step S3 is repeated. Therefore, a change of "the value indicating the distance" until the predetermined period of time has elapsed can be evaluated, so that it is possible to judge "whether the reciprocating action (a rise and a drop) of the bottom of bladder 112 that is performed at the speed equal to or higher than the predetermined moving speed within the predetermined period of time and represented by the value exceeding the certain value is performed or not".

For example, when the state as in Fig. 5 is changed to the state as in Fig. 6 after a first period of time has elapsed, a difference between the period of time Tl and the period of time Tt represents a "value indicating the moving distance" of the bottom of bladder 112 at the time when the state in Fig. 5 (where the pelvic floor muscles are loosened) is changed to the state in Fig. 6 (where the pelvic floor muscles are tensed). A value obtained by dividing the "value indicating the moving distance" by the "first period of time" is a value indicating the moving speed. As described above, if at least two waveforms can be acquired, the "value indicating the moving distance" of the bottom of bladder 112 and the "value indicating the moving speed" can be acquired. The predetermined period of time is ten minutes or shorter, desirably two minutes or shorter, and more desirably one minute or shorter. In a case where only the moving distance or the moving speed is measured, the predetermined period of time is desirably 30 seconds or shorter. The predetermined period of time is appropriately set in accordance with intended content of the judgment.

If it is judged in step S6 that the "predetermined motion" is performed, the controller 30 performs a first notification in step S7. If it is judged in step S6 that the "predetermined motion" is not performed, the controller 30 performs a second notification in step S8. As the second notification, for example, information indicating that "the predetermined motion (the predetermined movement of the bottom of bladder 112) is not performed" may be displayed or output by sound by the notification part 34 or may be transmitted to the external apparatus 190 by the communication part 32. As the first notification, for example, information indicating that "the predetermined motion (the predetermined movement of the bottom of bladder 112) is performed" may be displayed or output by sound by the notification part 34 or may be transmitted to the external apparatus 190 by the communication part 32. Alternatively, as the first notification, an index indicating the moving state of the bottom of bladder 112 may be displayed or output by sound by the notification part 34 or may be transmitted to the external apparatus 190 by the communication part 32. Examples of the index indicating the moving state of the bottom of bladder 112 includes, for example: a moving distance or a moving speed at the time when the bottom of bladder 112 rises, a moving distance or a moving speed at the time when the bottom of bladder 112 drops, a moving distance or a moving speed of a reciprocating action (a rise represented by the value exceeding the certain value and a drop represented by the value exceeding the certain value), the maximum moving distance (such as the maximum rise distance, the maximum drop distance, or the maximum moving distance in the reciprocating action) within the predetermined period of time, an average moving distance, the maximum moving speed, an average moving speed, the count of the reciprocating actions performed within the predetermined period of time, the counts of rising actions and dropping actions performed within the predetermined period of time, a period of time in which the moving state of the bottom of bladder is maintained, and the like. Examples of the period of time in which the moving state of the bottom of bladder is maintained includes, for example, a period of time, when a rise represented by a value exceeding a certain value occurs, in which a moving distance from a rising start position is maintained in a range exceeding the certain value (a period of time in which the bottom of bladder is maintained at a rising position).

The controller 30 corresponds to an example of a detection part and detects the "predetermined movement" of the bottom of bladder 112 and the "index indicating the moving state of the bottom of bladder 112" based on the result of the reception by the reception part. The "predetermined movement" of the bottom of bladder 112 is specifically, "a movement performed within a predetermined period of time by a moving distance equal to or exceeding a threshold", and in the example described above, is the predetermined motion (the reciprocating action of the bottom of bladder 112 represented by the value exceeding the certain value at the speed equal to or higher than the predetermined moving speed within the predetermined period of time).

The controller 30, the communication part 32, and the notification part 34 function as an example of an output part, and output information indicating the result of the detection by the detection part. The controller 30 and the communication part 32 correspond to an example of a transmission part, and operate to transmit, to the external apparatus 190, the information (such as the count of occurrences of the predetermined movement, the moving distance of the bottom of bladder 112, the moving speed of the bottom of bladder 112, and whether or not the predetermined movement has occurred). The external apparatus 190 may output the information by sound, displaying, or the like. The controller 30 and the notification part 34 output, by at least one of sound output or displaying, the information (such as the count of occurrences of the predetermined movement, the moving distance of the bottom of bladder 112, the moving speed of the bottom of bladder 112, and whether or not the predetermined movement has occurred). Since the notification part 34 is provided in the attaching unit 12, the information can be output from the attaching unit 12 near the subject 100.

### 1-3. Examples of Effects

The monitoring device 10 is capable of emitting the ultrasonic wave to the bottom of bladder 112 and detecting the predetermined movement of the bottom of bladder 112 or detecting the index indicating the moving state of the bottom of bladder 112. The monitoring device 10 does not focus on and monitor only the inflation or the shrinkage of the bladder 110 as a whole, but can focus on the bottom of bladder 112 and monitor the movement of the bottom of bladder 112. Therefore, even in a short period, when the bottom of bladder 112 moves, the monitoring device 10 can easily evaluate the movement more accurately. The monitoring device 10 is thus useful in verifying a state of a motion near the pelvic floor muscles 120.

The monitoring device 10 is particularly useful when being used in a scene where it is desired to promote the exercise of the pelvic floor muscles 120. For example, in a case where the exercise for pelvic floor muscle is done at a hospital, a rehabilitation facility, a nursing facility, a day care facility, a maternity clinic, a gym, and the like, it is useful for the exerciser to use the monitoring device 10. In this case, information obtained by the monitoring device 10 can be used by the exerciser, an instructor, an assistant, and other persons.

Since the monitoring device 10 has the output part that outputs the information indicating the result of the detection by the detection part, the information regarding the evaluation of the movement of the bottom of bladder 112 becomes available, and convenience is further enhanced. As the information indicating the result of the detection, information resulting from judgement based on the result of the detection may be displayed. For example, the output part may output information indicating the result of t information that indicates whether the subject is doing exercise successfully and appropriately, which is judged based on the result of the detection.

The monitoring device 10 outputs information including at least one of the following: "a count of occurrences of the predetermined movement", "a moving distance of the bottom of bladder 112", "a moving speed of the bottom of bladder 112", and "whether or not the predetermined movement has occurred". As a result, the moving state of the bottom of bladder 112 is evaluated by objective reference, and the evaluation information thereof can be used effectively.

The transmission part of the monitoring device 10 is capable of transmitting the information to the external apparatus 190. As a result, the information can be used effectively in the external apparatus 190.

In the monitoring device 10, the notification part 34 is provided in the attaching unit 12 to be attached to the subject 100, and the notification part 34 outputs the information by at least one of sound output or displaying. As a result, the subject 100 can recognize the information more easily.

### 2. Second Embodiment

### 2-1. Features of Monitoring Device 210

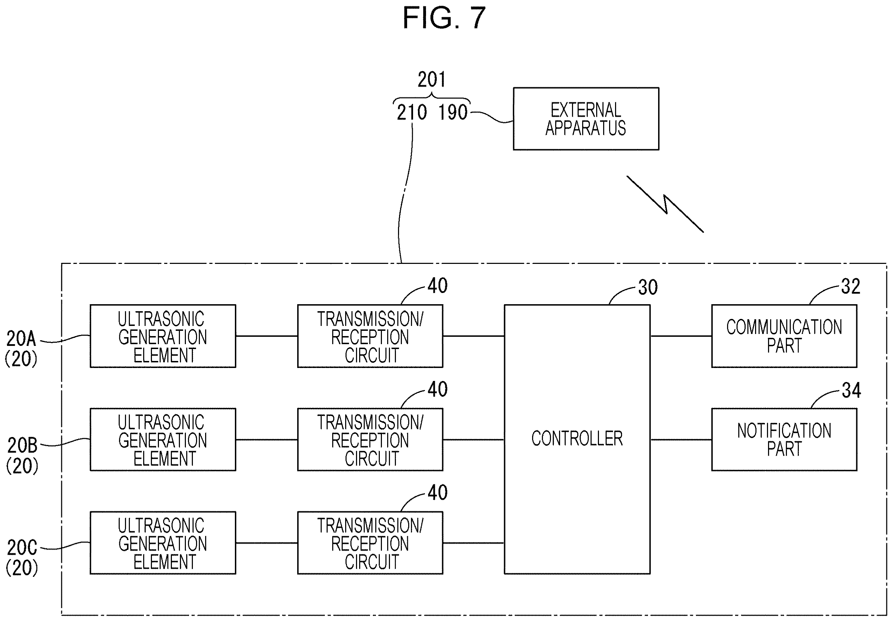

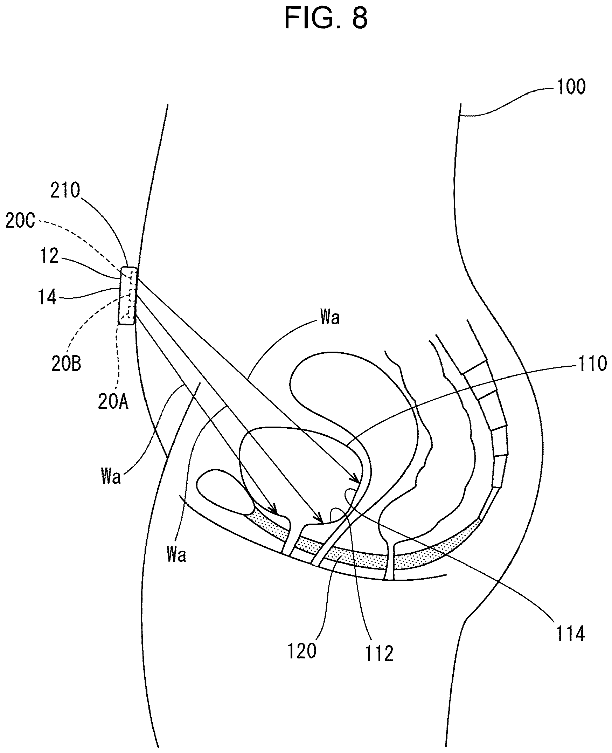

A monitoring device 210 of a second embodiment illustrated in Figs. 7 and 8 includes a plurality of ultrasonic generation elements 20 and a plurality of transmission/reception circuits 40. A monitoring system 201 has the same electrical configuration as that of the monitoring system 1 except that the plurality of ultrasonic generation elements 20 and the plurality of transmission/reception circuits 40 are provided. The hardware configuration and the basic operations of the controller 30, the communication part 32, the notification part 34, and the external apparatus 190 are the same as those of the monitoring system 1 illustrated in Fig. 1 and the like.

The ultrasonic generation elements 20 (a first element 20A, a second element 20B, and a third element 20C) of the monitoring device 210 each have the same configuration as that of the ultrasonic generation element 20 of the monitoring device 10 of the first embodiment. The transmission/reception circuits 40 each have the same configuration as that of the transmission/reception circuit 40 of the monitoring device 10 of the first embodiment. In the monitoring device 210, each ultrasonic generation element 20 can emit an ultrasonic wave and receive a reflected wave generated from the reflection of the ultrasonic wave on an object.

In the example in Fig. 8, the first element 20A and the second element 20B are disposed such that an ultrasonic wave from the first element 20A and an ultrasonic wave from the second element 20B are emitted to the bottom of bladder 112. The third element 20C is disposed such that an ultrasonic wave from the third element 20C is emitted to an inner side wall of bladder 114.

In the example in Fig. 8, the control as illustrated in Fig. 4 may also be performed. In this example, by the same method as that in the first embodiment, the value representing the distance to the bottom of bladder 112 is detected based on the waveform of the signal received by the first element 20A, and based on the result of the detection, whether the "predetermined motion" is performed or not can be judged in step S6 by the same method as that in the first embodiment. Likewise, by the same method as that in the first embodiment, the value indicating the distance to the bottom of bladder 112 is detected based on the waveform of the signal received by the second element 20B, and based on the result of the detection, whether the "predetermined motion" is performed or not can be judged in step S6 by the same method as that in the first embodiment. In this example, if the "predetermined motion" is confirmed based on the waveform of the signal received by any of the first element 20A, the second element 20B, and the third element 20C, the processing may proceed to Yes in step S6, and the first notification may be performed in step S7. If the "predetermined motion" is not confirmed based on the waveform of the signal received by any of the first element 20A, the second element 20B, and the third element 20C, the processing may proceed to No in step S6, and the second notification may be performed in step S8.

In the example in Figs. 7 and 8, a distance to the inner side wall of bladder 114 can also be detected based on the waveform of the signal received by the third element 20C. Furthermore, a change in the position of the inner side wall of bladder 114 in the predetermined period of time (a change in a distance from the monitoring device 210) and a moving distance can also be detected.

### 2-2. Examples of Effects

In the monitoring device 210 of the second embodiment, the emitting part includes the plurality of ultrasonic generation elements 20 (the first element 20A, the second element 20B, and the third element 20C). When ultrasonic waves are emitted to the bladder 110 of the subject 100 from two or more of the plurality of ultrasonic generation elements 20, and any of the ultrasonic waves is emitted to the bottom of bladder 112, the reception part can receive the reflected waves from the bladder 110 based on the respective ultrasonic waves. Since the monitoring device 210 can receive and use not only one reflected wave from the bottom of bladder 112 but also another reflected wave from the bladder 110, evaluation can be performed from more various angles, and the movement of the bottom of bladder 112 is likely to be evaluated more accurately.

In the monitoring device 210, when the emitting part (the plurality of ultrasonic generation elements 20) is disposed in the predetermined positional relationship with the subject 100, the emitting part emits the ultrasonic waves to the bottom of bladder 112 and the inner side wall of bladder 114 of the subject 100. In this case, the reception part (the plurality of ultrasonic generation elements 20) receives the reflected waves from the bottom of bladder 112 and the inner side wall of bladder 114 based on the respective ultrasonic waves. With the configuration as described above, the monitoring device 210 can evaluate not only the movement of the bottom of bladder 112 but also the movement of the inner side wall of bladder 114. With the monitoring device 210 as described above, for example, "a movement such that the bottom of bladder 112 moves largely the movement of the inner side wall of bladder 114 is little or there is no movement of the inner side wall of bladder 114 " or the like can also be verified. It is thus considerably useful in evaluating a state of a motion near the pelvic floor muscles 120 more accurately.

In the monitoring device 210, when the emitting part (the plurality of ultrasonic generation elements 20) is disposed in the predetermined positional relationship with the subject 100, the emitting part emits the ultrasonic waves to the bottom of bladder 112 of the subject 100. The reception part (the plurality of ultrasonic generation elements 20) receives the reflected waves from the bottom of bladder 112 based on the respective ultrasonic waves. With the configuration as described above, the monitoring device 210 can evaluate the movement of the bottom of bladder 112 more accurately by using a plurality of reflected waves. For example, in the case of the occurrence of a detection malfunction such that any of the reflected waves does not accurately reflect the movement of the bottom of bladder 112, it is possible to enhance the accuracy in evaluating the movement of the bottom of bladder 112 by using other reflected waves. Alternatively, if there is a difference in movement between the parts generating the reflected waves, information on the movement of each part can be collected and evaluated.

### <Other Embodiments>

The present disclosure is not limited to the embodiments described with the above description and the drawings. For example, any combination of the features of the embodiments described above or to be described later may be made as long as the combination is not inconsistent. Any of the features of the embodiments described above or to be described later may be omitted unless otherwise clearly stated as an essential feature. Further, the embodiments described above may be changed as follows.

In the embodiments above, a common ultrasonic generation element is configured as the emitting part and the reception part, but it is not limited to this example. In each example, the piezo-electric element configured as the emitting part may be different from a piezo-electric element configured as the reception part.

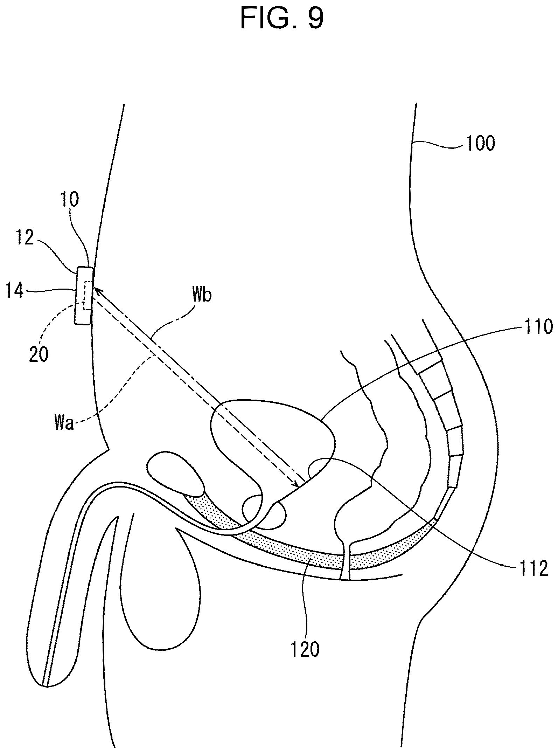

In the embodiments above, the case where the subject is a woman is exemplified; however, the subject may be a man as illustrated in Fig. 9. The example in Fig. 9 is different from the first embodiment in that the subject is a man, but other configurations except this are the same as those of the first embodiment.

In the first embodiment, it is judged in step S6 "whether the reciprocating action (a rise and a drop) of the bottom of bladder 112 that is performed at the speed equal to or higher than the predetermined moving speed within the predetermined period of time and represented by the value exceeding the certain value is performed or not". Instead of this, however, it may be judged in step S6 "whether the reciprocating action (a rise and a drop) of the bottom of bladder 112 that is performed at the speed equal to or higher than the predetermined moving speed within the predetermined period of time and represented by the value exceeding the certain value is performed a predetermined number of times or not".

In the examples in Figs. 5 and 6, the range corresponding to the bladder 110 may be defined in the following manner. First, the integral value of the entire section including up to the reflected waveform R1 is calculated. Then, the entire section is divided into any number of sections, and the integral value of the entire section is averaged by the number of sections to obtain the average value. If a certain range shows an integral value less than or equal to a predetermined proportion (for example, 10%) of the average value and is a maximum continuous range, the range may be determined as the range corresponding to the bladder.

The embodiments disclosed herein are to be construed as being illustrative and not restrictive in all aspects. It is intended that the scope of the present invention defined by the scope of claims, not be limited to the embodiments disclosed herein, and include the meaning equivalent to the scope of claims and any change made within the scope.

### Reference Signs List

10, 210 monitoring device
12 attaching unit
20 ultrasonic generation element (emitting part, reception part)
30 controller (detection part, output part)
32 communication part (output part)
34 notification part (output part)
100 subject
110 bladder
112 bottom of bladder
114 inner side wall of bladder
190 external apparatus

## Claims

1. A monitoring device having an emitting part that emits an ultrasonic wave into a body of a subject, the monitoring device comprising:
a reception part that receives a reflected wave based on the ultrasonic wave reflected on a bottom of bladder of the subject; and
a detection part that detects, based on a result of reception by the reception part, a predetermined movement of the bottom of bladder or an index indicating a moving state of the bottom of bladder.

2. The monitoring device according to claim 1, comprising:
an output part that outputs information indicating a result of detection by the detection part.

3. The monitoring device according to claim 2,
wherein the information includes at least one of the following: a count of occurrence of the predetermined movement, a moving distance of the bottom of bladder; a moving speed of the bottom of bladder; whether or not the predetermined movement has occurred; and a period of time in which the moving state of the bottom of bladder is maintained.

4. The monitoring device according to claim 2 or 3,
wherein the output part includes a transmission part that transmits the information to an external apparatus.

5. The monitoring device according to any one of claims 2 to 4, comprising:
an attaching unit to be attached to the subject,
wherein the output part includes a notification part included in the attaching unit, and
wherein the notification part outputs the information by at least one of sound output or displaying.

6. The monitoring device according to any one of claims 1 to 5, wherein
the emitting part includes a plurality of ultrasonic generation elements, and
when ultrasonic waves are emitted from two or more ultrasonic generation elements included in the plurality of ultrasonic generation elements to a bladder of the subject, and any of the ultrasonic waves is emitted to the bottom of bladder, the reception part receives reflected waves from the bladder based on the respective ultrasonic waves.

7. The monitoring device according to claim 6, wherein
the emitting part is configured to emit the ultrasonic waves to the bottom of bladder and an inner side wall of bladder of the subject when the emitting part is disposed in a predetermined positional relationship with the subject, and
the reception part is configured to receive reflected waves from the bottom of bladder and from the inner side wall of bladder based on the respective ultrasonic waves.

8. The monitoring device according to claim 6, wherein
the emitting part is configured to emit the ultrasonic waves to the bottom of bladder of the subject when the emitting part is disposed in a predetermined positional relationship with the subject, and
the reception part is configured to receive reflected waves from the bottom of bladder based on the respective ultrasonic waves.
